# FASCICULE DE BREVET EUROPEEN

(11) **EP 1 082 968 B1**
(45) Date de publication et mention de la délivrance du brevet: **11.05.2005**
(21) Numéro de dépôt: 00402472.5
(22) Date de dépôt: 08.09.2000
(51) Int. Cl.: A61L 9/12, B60H 3/00

(54) **Agencement pour parfumer l'intérieur d'un habitacle de véhicule automobile**
Parfümiervorrichtung für den Fahrzeuginnenraum
Device for perfuming a motor vehicle interior

(30) Priorité: 10.09.1999 FR 9911330
(43) Date de publication de la demande: 14.03.2001
(73) Titulaire: Renault s.a.s., 92100 Boulogne Billancourt (FR)
(72) Inventeur: Nesa, Daniel, 91430 Igny (FR); Couderc, Stéphane, 91160 Champlan (FR)

(56) Documents cités:
- WO-A-88/05731
- GB-A- 2 158 715
- US-A- 5 314 669

## Description

L'invention concerne un agencement pour parfumer l'intérieur d'un habitacle de véhicule automobile.

L'invention concerne plus particulièrement un agencement pour parfumer l'intérieur d'un habitacle de véhicule automobile, du type dans lequel l'habitacle du véhicule est délimité par une caisse qui comporte au moins un élément de structure de caisse tubulaire creux dont au moins une ouverture débouche à l'intérieur de l'habitacle.

On connaît de nombreux exemples d'agencements destinés à parfumer l'intérieur d'un habitacle de véhicule automobile.

Il s'agit pour la plupart d'agencements comportant des substances odorantes, initialement contenues dans des bombes ou dans des diffuseurs, qui sont pulvérisées par l'utitisateur ou diffusées en différent points de l'habitacle du véhicule pour lui conférer une odeur agréable correspondant au désir de l'utilisateur.

Ces substances odorantes ont pour inconvénient de ne présenter qu'un effet limité dans le temps, de risquer de tacher les vêtements des passagers, et, dans le cas des parfums contenus dans des diffuseurs, de nécessiter la présence de diffuseurs souvent peu esthétiques à l'intérieur du véhicule.

On connaît aussi des agencements dans lesquels des éléments parfumés, tels que des cartons disposés sous les garnitures des sièges du véhicule, ou des éléments d'aménagement intérieur du véhicule en matériau plastique parfumé, sont montés en usine lors de la fabrication du véhicule.

Ces agencements présentent eux aussi une efficacité limitée dans le temps, et sont de plus onéreux, car ils doivent être adaptés à chaque type particulier de véhicule.

Or, on a constaté que l'utilisation de cires anti-corrosion pulvérisée dans des éléments de structure de caisse tubulaire creux de l'habitacle du véhicule par des ouvertures de ces éléments laissait perdurer un certain temps une odeur tenace dans celui-ci, qui se répandait dans l'habitacle par ces ouvertures.

Selon les goûts de l'utilisateur, cette odeur de durée limitée peut-être appréciée et même recherchée comme un témoignage de l'état neuf du véhicule, ou au contraire être ressentie comme une géne sans qu'il soit possible de modifier cette odeur au gré et à la convenance de l'utilisateur avant un long moment.

Pour remédier aux inconvénients précités l'invention propose d'utiliser les éléments de structure de caisse tubulaire creux de l'habitacle du véhicule comme supports pour un agent odorant convenant à l'utilisateur.

Dans ce but, l'invention propose un agencement du type décrit précédemment, caractérisé en ce qu'un agent odorant est disposé à l'intérieur de l'élément de structure creux pour diffuser une odeur déterminée à l'intérieur de l'habitacle du véhicule.

Selon d'autres caractéristiques de l'invention :
- l'agent odorant est contenu dans une cire anti-corrosion, initialement liquide, qui est introduite par pulvérisation dans l'ouverture de l'élément de structure de caisse creux, lors d'une opération de traitement anti-corrosion de la caisse du véhicule,
- l'agent odorant est contenu dans un support formant tampon d'insonorisation qui est reçu à l'intérieur de l'élément de structure de caisse creux,
- le support formant tampon est introduit dans l'élément de structure de caisse creux par l'ouverture,
- le support formant tampon est disposé dans l'élément de structure lors d'une opération d'assemblage de caisse dans laquelle au moins deux parties ouvertes de l'élément de structure de caisse sont assemblées l'une à l'autre pour former l'élément de structure de caisse tubulaire,
- le support formant tampon est constitué d'une mousse dont des cellules ouvertes reçoivent l'agent odorant,
- le support formant tampon est réalisé dans un matériau plastique rigide dont l'agent odorant est un composant,
- l'élément tubulaire creux constitue un conduit dans lequel circule un flux d'air de climatisation ou de chauffage de l'habitacle.

D'autres caractéristiques et avantages de l'invention apparaîtront à la lecture de la description détaillée qui suit-pour la compréhension de laquelle on se reportera aux dessins annexés dans lesquels :
- la figure 1 est une vue en perspective d'un élément de structure de caisse creux d'une caisse de véhicule automobile recouvert intérieurement d'une cire odorante parfumée selon un premier mode de réalisation de l'invention,
- la figure 2 est une vue en perspective d'un élément de structure de caisse creux d'une caisse de véhicule automobile comportant un support formant tampon constitué d'une mousse parfumée selon un deuxième mode de réalisation de l'invention,
- la figure 3 est une vue en perspective d'un élément de structure de caisse creux d'une caisse de véhicule automobile comportant un support réalisé dans un matériau plastique rigide parfumé selon un troisième mode de réalisation de l'invention.
- la figure 4 est une vue en perspective éclatée illustrant le montage d'un élément de structure de caisse creux en deux parties d'une caisse de véhicule automobile et la mise en place d'un support selon les deuxième ou troisième mode de réalisation de l'invention.

Dans la description qui va suivre, des chiffres de référence identiques désignent des pièces identiques ou ayant des fonctions similaires.

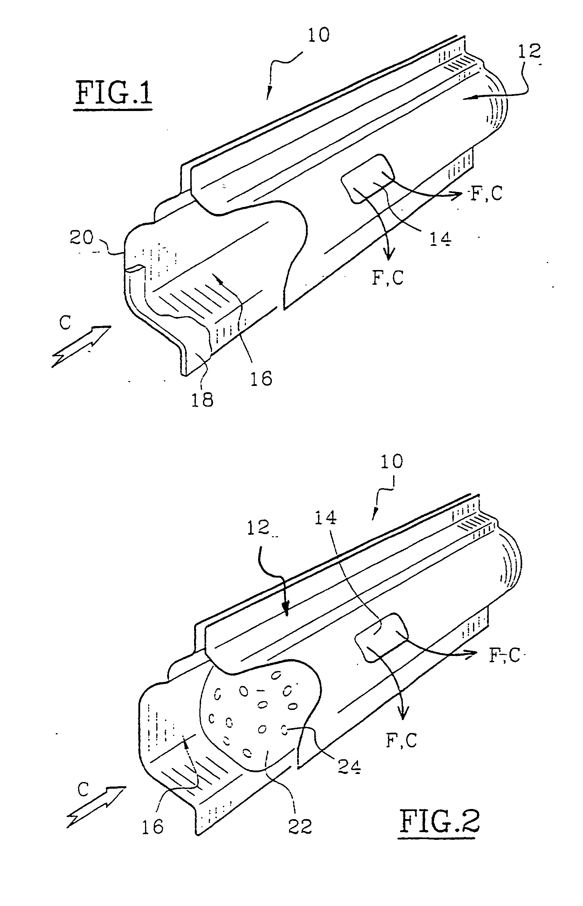

On a représenté à la figure 1 un agencement 10 pour parfumer l'intérieur d'un habitacle (non représenté) de véhicule automobile.

De manière connue, un habitacle de véhicule automobile comporte au moins un élément 12 de structure de caisse tubulaire creux qui est destinée à assurer la rigidité de l'habitacle et à assurer la protection des passagers du véhicule.

Cet élément 12 de structure de caisse comporte couramment, pour faciliter sa préhension, lors du processus de fabrication du véhicule, au moins une ouverture 14 qui débouche à l'intérieur de l'habitacle du véhicule.

Conformément à l'invention, un agent odorant est disposé à l'intérieur 16 de l'élément 12 de structure de caisse creux pour diffuser une odeur déterminée à l'intérieur de l'habitacle du véhicule.

On a représenté aux figures 1 à 4 un élément 12 de structure de caisse qui est formée d'un bavolet longitudinal formant de manière connue un bas de caisse pour l'habitacle du véhicule.

Selon un premier mode de réalisation de l'invention qui est représenté à la figure 1, le bavolet 12 est recouvert intérieurement d'une cire anticorrosion 18 qui est pulvérisée, lors de la fabrication de l'habitacle, par l'ouverture 14 de manière à recouvrir les parois 20 du bavolet 12 et assurer ainsi sa protection contre la corrosion. Dans le premier mode de réalisation de l'invention, la cire 18 contient l'agent odorant de sorte que l'odeur de celui-ci est susceptible de se diffuser à l'intérieur de l'habitacle du véhicule en s'échappant par l'ouverture 14 comme représenté par les flèches F de la figure 1.

Ce premier mode de réalisation est particulièrement avantageux car il permet, sans ajouter d'opération supplémentaire au processus de fabrication, de parfumer durablement l'intérieur de l'habitacle du véhicule.

Il est connu de disposer à l'intérieur d'un élément de structure tubulaire creux de l'habitacle du véhicule un support formant un tampon 22 d'insonorisation dont la fonction est de filtrer les bruits de roulement du véhicule.

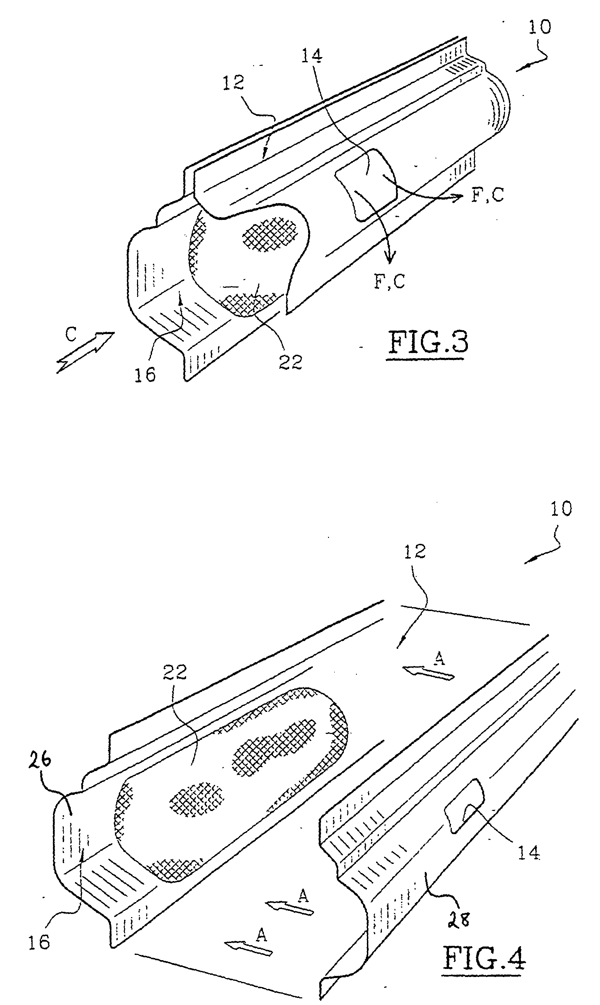

Comme l'illustre les figures 2 et 3, des deuxième et troisième modes de réalisation de l'invention consistent en ce que l'agent odorant est contenu dans le support formant tampon 22 d'insonorisation.

Ainsi, selon le deuxième mode de réalisation de l'invention, le tampon 22 d'insonorisation parfumé peut être constitué d'une mousse, notamment une mousse d'origine synthétique qui comporte des cellules 24 ouvertes qui contiennent l'agent odorant de façon que, d'une façon analogue au premier mode de réalisation de l'invention, son odeur soit diffusée vers l'intérieur de l'habitacle suivant les flèches F par l'ouverture 14 du bavolet 12.

Il sera compris que ce second mode de réalisation n'intervient pas nécessairement en remplacement du premier mode de réalisation de l'invention, mais peut constituer un complément efficace à la pulvérisation d'une cire anticorrosion parfumée de façon à renforcer le pouvoir odorant de l'agent odorant contenu dans le bavolet 12 ou l'élément de structure tubulaire creux du véhicule.

Dans ce mode de réalisation, lors de la fabrication du véhicule, le tampon 22 d'insonorisation parfumé peut-être un tampon 22 qui présente des caractéristiques de compressibilité suffisantes pour permettre son introduction par l'orifice 14, ou, en première variante, être constitué d'une mousse qui est introduite sous forme pâteuse dans le bavolet 12 et qui ne se vulcanise au contact de l'air qu'une fois à l'intérieur du bavolet 12, ou encore, en seconde variante, être disposé à l'intérieur du bavolet 12 avant l'assemblage de celui-ci, comme il sera décrit plus en détail en référence à la figure 4.

La figure 3 illustre un troisième mode de réalisation de l'invention dans lequel le tampon 22 d'insonorisation parfumé est réalisé dans un matériau plastique rigide dont l'agent odorant est un composant.

Avantageusement, cette configuration est d'une réalisation aisée car il est connu de longue date d'incorporer à la fabrication des matériaux plastiques, réalisés à base de produits pétroliers, des carbures aromatiques qui leur confèrent une odeur déterminée.

Il sera compris que ce troisième-mode de réalisation n'intervient pas nécessairement en remplacement du premier mode de réalisation de l'invention, mais peut aussi constituer un complément efficace à la pulvérisation d'une cire anticorrosion parfumée de façon à renforcer le pouvoir odorant de l'agent odorant contenu dans le bavolet 12 ou l'élément de structure tubulaire creux du véhicule.

Dans ce mode de réalisation, du fait de la rigidité du matériau plastique du tampon 22, seulement deux modes d'introduction du tampon 22 de intérieur du bavolet 12 sont possibles.

En effet, selon une première variante du troisième mode de réalisation, le bavolet 12 peut comporter une ouverture 14 de grande taille qui permet l'introduction, comme illustré par la flèche en pointillés, du tampon 22 à l'intérieur du bavolet 12.

Par ailleurs, selon une seconde variante du troisième mode de réalisation, le tampon 22 peut être disposé à l'intérieur du bavolet 12 avant l'assemblage de celui-ci, comme il sera décrit plus en détail en référence à la figure 4.

Comme l'illustrent les flèches C des figures 1, 2, et 3, le bavolet 12 ou l'élément creux tubulaire de structure qui en tient lieu peut avantageusement constituer un conduit dans lesquels circule un flux C d'air pulsé issu d'un circuit (non représenté) de climatisation ou de chauffage de l'habitacle. Cette configuration est particulièrement avantageuse car elle permet une diffusion accrue de l'odeur générée par l'agent odorant contenu dans le bavolet 12, le flux odorant C s'échappant par l'ouverture 14.

Avantageusement, pour chacun de ses modes de réalisation, il est possible de sélectionner lors de la fabrication du véhicule le parfum choisi généré par l'agent odorant disposé à l'intérieur du bavolet 12. De la sorte, ce parfum peut constituer un parfum typique propre à un constructeur automobile déterminé, c'est-à-dire une "signature olfactive" du constructeur, ou propre à une gamme déterminée de véhicules du constructeur automobile. Ce parfum peut aussi faire partie d'un éventail de parfums qui sont mis à la disposition du client lors de l'achat du véhicule en concession, et constituer un élément de personnalisation du véhicule au même titre que le choix d'un coloris de la caisse du véhicule ou d'un tissu de garnissage des sièges du véhicule.

Comme l'illustre la figure 4, le tampon 22 d'insonorisation peut, dans les deuxième et troisième modes de réalisation de l'invention être introduit dans l'élément tubulaire creux de structure de caisse ou bavolet 12 du véhicule préalablement à la fabrication effective de celui-ci.

En effet, il est connu de fabriquer un tel élément de structure de caisse en réunissant par soudage au moins deux parties 26 et 28 semi-tubulaires qui, une fois assemblées, forment l'élément tubulaire creux ou le bavolet 12, comme illustré par les flèches A de la figure 4.

Ainsi, le tampon 22 d'insonorisation parfumé réalisé en mousse ou en matériau plastique conformément au deuxième et troisième mode de réalisation de l'invention peut être placé dans une des parties 26 ou 28 semi-tubulaires avant que, comme l'illustrent les flèches évidées, n'y soit assemblée la partie complémentaire permettant de former l'élément creux ou bavolet 12.

L'agencement 10 selon l'invention permet donc pour l'utilisateur de disposer durablement d'un habitacle parfumé sans qu'il soit besoin d'y ajouter de dispositifs supplémentaires peu esthétiques.

## Revendications

1. Agencement (10) pour parfumer l'intérieur d'un habitacle de véhicule automobile, du type dans lequel l'habitacle du véhicule est délimité par une caisse qui comporte au moins un élément (12) de structure de caisse tubulaire creux dont au moins une ouverture (14) débouche à l'intérieur de l'habitacle,
**caractérisé en ce qu'**un agent odorant est disposé à l'intérieur de l'élément (12) de structure creux pour diffuser une odeur déterminée à l'intérieur de l'habitacle du véhicule.

2. Agencement (10) selon la revendication 1, **caractérisé en ce que** l'agent odorant est contenu dans une cire (18) anti-corrosion, initialement liquide, qui est introduite par pulvérisation dans l'ouverture (14) de l'élément de structure de caisse creux, lors d'une opération de traitement anti-corrosion de la caisse du véhicule.

3. Agencement (10) selon la revendication 1, **caractérisé en ce que** l'agent odorant est contenu dans un support (22) formant tampon d'insonorisation qui est reçu à l'intérieur de l'élément (12) de structure de caisse creux.

4. Agencement (10) selon la revendication 3, **caractérisé en ce que** le support (22) formant tampon est introduit dans l'élément (12) de structure de caisse creux par l'ouverture (14).

5. Agencement (10) selon la revendication 3 , **caractérisé en ce que** le support formant tampon (22) est disposé dans l'élément (12) de structure lors d'une opération d'assemblage de caisse dans laquelle au moins deux parties (26, 28) ouvertes de l'élément (12) de structure de caisse sont assemblées l'une à l'autre pour former l'élément (12) de structure de caisse tubulaire.

6. Agencement (10) selon les revendications 4 ou 5, **caractérisé en ce que** le support formant tampon (12) est constitué d'une mousse dont des cellules (24) ouvertes reçoivent l'agent odorant.

7. Agencement (10) selon les revendications 4 ou 5, **caractérisé en ce que** le support formant tampon (12) est réalisé dans un matériau plastique rigide dont l'agent odorant est un composant.

8. Agencement (10) selon l'une quelconque des revendications précédentes, **caractérisé en ce que** l'élément (12) tubulaire creux constitue un conduit dans lequel circule un flux (C) d'air de climatisation ou de chauffage de l'habitacle.

## Patentansprüche

1. Anordnung (10) zum Parfümieren des Inneren einer Fahrgastzelle eines Kraftfahrzeugs, von dem Typ, in dem die Fahrgastzelle des Fahrzeugs durch eine Karosserie begrenzt ist, die mindestens ein hohles, rohrförmiges Karosserie-Strukturelement (12) aufweist, von dem mindestens eine Öffnung (14) in das Innere der Fahrgastzelle mündet,
**dadurch gekennzeichnet, dass** ein wohlriechendes Mittel im Inneren des hohlen Strukturelements (12) angebracht wird, um einen bestimmten Geruch im Inneren der Fahrgastzelle zu verbreiten.

2. Anordnung (10) gemäß Anspruch 1, **dadurch gekennzeichnet, dass** das wohlriechende Mittel in einem Anti-Korrosions-Wachs (18) enthalten ist, das anfangs flüssig ist und das durch Zerstäubung in einer Öffnung (14) des hohlen Karosserie-Strukturelements bei einer Operation der Anti-Korrosions-Behandlung der Karosserie des Fahrzeugs eingebracht wird.

3. Anordnung (10) gemäß Anspruch 1, **dadurch gekennzeichnet, dass** das wohlriechende Mittel in einem Träger (22) enthalten ist, der einen Schalldämpfungspuffer bildet und der im Inneren des hohlen Karosserie-Strukturelements (12) empfangen wird.

4. Anordnung (10) gemäß Anspruch 3, **dadurch gekennzeichnet, dass** der Träger (22), der einen Puffer bildet, in das hohle Karosserie-Strukturelement (12) durch die Öffnung (14) eingeführt wird.

5. Anordnung (10) gemäß Anspruch 3, **dadurch gekennzeichnet, dass** der Träger (22), der einen Puffer bildet, im Strukturelement (12) bei einer Operation der Montage der Karosserie angeordnet wird, bei der mindestens zwei offene Teile (26, 28) des Karosserie-Strukturelements (12) montiert werden, das eine an das andere, um das rohrförmige Karosserie-Strukturelement (12) zu bilden.

6. Anordnung (10) gemäß Anspruch 4 oder 5, **dadurch gekennzeichnet, dass** der einen Träger bildende Puffer (12) aus einem Schaumstoff besteht, dessen offene Zellen (24) das wohlriechende Mittel empfangen.

7. Anordnung (10) gemäß Anspruch 4 oder 5, **dadurch gekennzeichnet, dass** der den Träger bildende Puffer (12) in einem steifen Kunststoffmaterial realisiert wird, bei dem das wohlriechende Mittel ein Bestandteil ist.

8. Anordnung (10) gemäß einem der vorherigen Ansprüche, **dadurch gekennzeichnet, dass** das hohle, rohrförmige Element (12) eine Leitung bildet, in der ein Luftfluss (C) zur Klimatisierung oder Heizung der Fahrgastzelle zirkuliert.

## Claims

1. An arrangement (10) for perfuming the interior of a passenger space of an automobile vehicle, of the type in which the vehicle passenger space is bounded by a bodywork which comprises at least one hollow tubular bodywork structure member (12), at least one opening (14) of which communicates with the passenger space, **characterised in that** an odorising agent is disposed in a hollow bodywork structure member (12) in order to diffuse a predetermined odour into the vehicle passenger space.

2. An arrangement (10) as claimed in claim 1, **characterised in that** the odorising agent is contained in an initially liquid anti-corrosion wax (18) which is introduced by spraying into the opening (14) of the hollow bodywork structure member during an operation to treat the vehicle bodywork against corrosion.

3. An arrangement (10) as claimed in claim 1, **characterised in that** the odorising agent is contained in a support (22) forming a sound-proofing buffer which is received within the hollow bodywork structure member (12).

4. An arrangement (10) as claimed in claim 3, **characterised in that** the support (22) forming a buffer is introduced into the hollow bodywork structure member (12) via the opening (14).

5. An arrangement (10) as claimed in claim 3, **characterised in that** the support forming a buffer (22) is disposed in the bodywork structure member (12) during a bodywork assembly operation in which at least two open portions (26, 28) of the bodywork structure member (12) are assembled with one another to form the tubular bodywork structure member (12).

6. An arrangement (10) as claimed in claims 4 or 5, **characterised in that** the support forming a buffer (22) is formed by a foam whose open cells (24) receive the odorising agent.

7. An arrangement (10) as claimed in claims 4 or 5, **characterised in that** the support forming a buffer (22) is made from a rigid plastics material of which the odorising agent is a component.

8. An arrangement (10) as claimed in any one of the preceding claims, **characterised in that** the hollow tubular member (12) forms a duct in which a flow (C) of conditioning or heating air for the passenger space circulates.
